**Europäisches Patentamt**

**European Patent Office**

**Office européen des brevets**

(19)

(11) **EP 1 118 859 A2**

(12) # EUROPEAN PATENT APPLICATION

(43) Date of publication:
**25.07.2001 Bulletin 2001/30**

(51) Int Cl.7: **G01N 33/52**, G01N 33/487, G01N 21/25

(21) Application number: **01101270.5**

(22) Date of filing: **19.01.2001**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **21.01.2000 JP 2000013114**
**17.05.2000 JP 2000145436**
**10.08.2000 JP 2000242915**
**25.08.2000 JP 2000254743**

(71) Applicant: **WAKO PURE CHEMICAL INDUSTRIES, LTD**
**Chuo-ku Osaka (JP)**

(72) Inventors:
• **Iwata, Kenji, c/o Wako Pure Chemical Industries**
**Amagasaki-shi, Hyogo (JP)**

• **Hamanaka, Tadashi**
**Amagasaki-shi, Hyogo (JP)**
• **Hatayama, Yasumichi**
**Amagasaki-shi, Hyogo (JP)**
• **Wada, Shogo**
**Amagasaki-shi, Hyogo (JP)**

(74) Representative: **Schirdewahn, Jürgen**
**Elisabeth Jung, Dr. Phil., Dipl.-Chem,**
**Jürgen Schirdewahn, Dr. rer.nat., Dipl.-Phys.,**
**Claus Gernhardt, Dipl.-Ing.,**
**Patentanwälte,**
**Clemensstrasse 30**
**80803 München (DE)**

(54) **A test device for a multi-items test and the method for producing the same as well as measuring instrument for the test device**

(57) The present invention is to provide a test device for a multi-items where in all the test papers for all items for one test are wetted by one shot dropping and transportation of a detecting part (module) or a test device is not required upon measurement. Also, the present invention has the object to provide an analyzer for a test device which makes it possible as in the known visual tests to conduct measurement easily while the test device is kept on a cup and thus can be used in bed side medical examinations and regional medical examinations with no considerable problem.

F I G. 1

**Description**

BACKGROUND OF THE INVENTION

Field of the Invention

The present invention relates to a test device for a multi-items test of components in a sample originating from a living body, which is equipped with micro test papers used in hospitals, commercial laboratories and the like, more concretely a test device for a multi-items test useful as urine test devices, microorganism test devices, blood or serum test devices for automatic analyzers, and also to a method for preparation of the test device. The present invention also relates to an analyzer for a test device in which components in a sample can be measured accurately only by positioning reading parts for the test device opposite to the test device to be measured, more particularly, relates to an analyzer for a test device in which the measuring parts are constructed in such a way as capable of freely transporting by hand (carrying) and the measurement is possible even at bed side.

Background of the Invention

As test devices for automatic urine analyzers, use has so far widely been made of stick type urine test devices wherein multiple numbers of test papers each of which carries reagents for each test item are fixed at predetermined intervals on a stick made of plastics.

In this stick type device for test of urine, each one test device is used, in principle, for one test per one sample and measurement is conducted by a dip (and read) method or a dropping method, but there have been observed such drawbacks that dipping is difficult if an amount of a sample is small and measurement becomes inaccurate by interaction influences among the items to be measured because plural items are generally measured by reactions on one and the same supporting material.

In order to solve those drawbacks, the present patent applicant has developed a test device wherein plural numbers of test papers are placed on bottoms of concave portions separately among papers for each of items to be tested and the open parts of the concave portions are covered with a mesh material so as to prohibit cross-contaminations among the items, and has filed a patent application for this invention (Japanese Patent Publication-Kokai- No. 14623/1999).

The above device is an epoch-making one from a view point of prohibiting cross-contaminations, but has such problems that when the numbers of the items to be tested are large, a large amount of a sample is required and/or a long time is required for the tests.

Namely, when the numbers of the items to be tested are increased in a dip (and read) method, a large amount of a sample is required because deep dipping of test devices is necessary, and when the numbers are increased in a dropping method, a long time is required for the tests because each of the tests has been conducted by dropping the sample one by one.

Further, in known device for the tests, there has been caused such a problem that when the tests are conducted by a measurement instrument, each of the items is tested on each test paper corresponding to the items with mechanically moving the test device or a light source-measurement part, and therefore a long time is required for the tests.

As a microorganism test device used for identification of microorganism, there has been heretofore generally used a plastic plate having a plurality of wells in which reagents such as media and various reaction substrate are sealed as powder or are air-dried, or a disk formed by an absorptive carrier such as a filter paper impregnated with the media and/or reagents.

In such a microorganism test device, measurement is normally done visually (by eyes). Since, therefore, it has the size necessary for that purpose, if the number of items is increased, much quantity of sample is required. Further, there is a problem that it takes much time for measurement.

As test devices for clinical automatic analyzer with dry chemistry, use has been made of those as roughly classified into the following three categories.

(1) Slide type test device composed of a porous dispersion layer, a reagent layer and a transparent plastic film, which are laminated in this order from the upper to the bottom.
(2) Stick type test device wherein a multi-layered test paper carrying reagents for each items to be tested is fixed on a plastic film or plural number of the multi-layered test papers are fixed thereon at a suitable interval.
(3) Stick type test device for testing one item, similarly to the just above (2), wherein a sample is developed from a dropping part and a reaction is allowed to take place on a reagent layer set forth at another position to determine the object.

In the above test devices of (1) to (3), each item to be tested is in each independent situation, and therefore in case of measurement of plural numbers of items, it is required to mechanically transport a distributor or a measurement instrument or alternately the test device itself upon distribution and measuring of sample. For this reason, there has been a problem of requirement of a long time for measurement, because a moving part is necessary for measurement of plural numbers of items, which causes requirement of a large instrument and much time for movement.

Additionally, an area for tests becomes large and sliding frame parts and wrapping materials are independently required, which causes high cost for the test device. This has been a great barrier for further spreading a biochemical automatic analyzer by dry chemistry.

In a case where concentrations of components in an urine, a blood sample such as blood, serum and plasma, etc. are measured by using the test device or a kind of a microorganism in s sample is identified by the test device, it has so far been conducted to measure a degree of coloring in a reagent layer or a change of color in a culture solution of a microorganism by visual means or by a photo-analyzer using a reflection meter.

Such a instrument as above includes one described in Japanese Patent Publication-Kokai- No. 31011/1998 and No. 43387/1996.

Such known instruments as above are large in size and thus impossible to carry about easily and the test device has to be held on reading parts of the test device. The reason is that distances from a detector in each of measurements can be kept constant by holding the test device on a specified position.

The known instrument as mentioned above have made it difficult to transport the measuring parts, it is not possible to measure urine and blood at the bed side as they are not portable type, but fixed type.

Further urine tests in regional medical examinations have almost been conducted by visual method (eyes), and from this point of view, mechanization has still remarkably been behindhand. This is because collations of testees, samples and data among them is necessary and for this reason it takes much time for the test, while in measurements by visual method the test result can be obtained only by immersing a test device in urine cup in the testee's presence, followed by keeping for 10 to 30 seconds and thus the test can be conducted in such an effective way as conducting about 10 person's tests at one time in parallel.

Further, in the test for identification of microorganism, an automatic apparatus comprising modules such as sampler, incubator, computer, printer, etc. is used.

Such an apparatus as described above requires an exclusive-use test device, lacking in generality. Further, for the reasons that the apparatus itself is large and expensive, the apparatus is not widely used, and most of measurements are still being done visually (by eyes) in the existing circumstances.

SUMMARY OF THE INVENTION

It is an object of the present invention to provide a test device where sample can be dropped in or adhered to all the test papers for all items for the test by one shot dropping and mechanical transportation of a detecting part (module) or a test device is not required upon measurement.

The present invention also has the object of providing a device for a multi-items test which can prohibit cross-contaminations and test many items with the use of a small amount of a sample for a short period of time.

The present invention also has the object to provide a test device for a multi-items wherein mechanical transportation of a distributor, a detecting part (module) or a test device is not required upon distributions and measurement so that measurement can be conducted in short time and the analyzer can be made into compact and produced at low cost.

Further, the present invention has the object to provide a test device of making it possible to distribute a sample to test papers for all items to be tested by dropping a sample to only one position.

The present invention in also has the object to provide a test device for measurement of components in a sample originating from a living body which makes a sample easily impregnated which results in short measurement time.

Also, the present invention has the object to provide a method for preparation of above-mentioned test device easily and at low cost.

Further the present invention has the object to provide an analyzer for a test device which makes it possible as in the known visual tests to conduct measurement easily while the test device is kept on a cup and thus can be used at bed side upon medical examinations and regional medical examinations with no considerable problem.

The present inventors have extensively made study in order to attain the above object to arrive at an idea that by making a size of test paper remarkably small (micro test paper), a sample can be dropped or distributed (adhered) to test papers for all items to be tested by one shot dropping and further measurement can be conducted without mechanical transportation of the detecting instrument or the test paper, and the invention has been accomplished on the basis of this idea.

And, there has been put into a market no such test device in which a sample can be dropped or adhered to test papers for all items to be tested by only one shot dropping from only one and the same distributor, and measurement can be conducted without mechanical transportation of a detecting part or the test device, and there has never been known such an idea as above.

Namely, the test device of the present invention is characterized in that micro test papers for a multi-items test of a sample, characterized in that micro test papers for the multi-items test are held on the bottoms of concave portions in the number required for the multi-items test for one test per the sample, the concave portions being set forth separately with one another by divider walls in such a way that all the test papers for all items for one test are wet by one shot dropping of the sample, and the depth of the concave portions being more than a thickness of the micro test papers.

The method for preparation a test device of the present invention is characterized in that a process of adhering a second sheet material formed with a number of through-holes to a first sheet material and a process of adhering a micro test paper to said hole or a part at which said hole is positioned before or after said second sheet material is adhered, said hole having the depth greater than the thickness of said test paper.

In the present invention, because making the test papers small in size, by a single dropping of a sample to each of the papers, all papers can be wetted.

Alternately, the test papers are placed in each of concave portions for one test which are connected to a concave portion for dropping of a sample through ditches and the sample is dropped in the dropping part, whereby the sample is flown into the concave portions for one test through the ditches to wet the test papers, and still alternately the test papers are placed in each of concave portions for one test which are separately formed and the sample is dropped in each of the concave portions for one test through a dropping instrument equipped with plural numbers of distributors (wherein the sample is dropped to the test papers through the distributors).

In the present invention, the micro test paper means a test paper of such a considerably small size as hardly capable of determining the test result visually (by eyes) or not suitable for visual determination of the test result.

More concretely, the size of the micro test papers is such as capable of allowing all multi-items test papers for one test per one sample to be wet by one shot of drop of the sample, more definitely 0.5 to 3.0 mm of a diameter or a length between the two opposite sides of the micro test paper.

And, the urine test paper or the microorganism test paper is used for visual determination (or capable of determining even visually), and therefore some degree of a size is necessary for these paper. Under the situation, it has so far never been contrived to reduce its size to such small as the micro test paper in which visual determination is difficult or hardly possible.

It has been considered that a test paper should be considerable large size for conducting determination by using a test device for dry chemistry, but the extensive study by the present inventors has found that the object measurement can be conducted at high accuracy by using the micro test paper having such a size as in the present invention in combination with the most recent optical technologies (such as CCD camera) and a instrument using trace amount distribution technology, and the present invention has been accomplished based upon this finding.

The present invention relates to an analyzer for detecting components of a sample in a measuring test device which is composed of measuring parts equipped with a detecting part for measuring reflection lights and control parts set forth integrated in or separately from the measuring parts, which is characterized in that there are set forth parts for reading the measuring test device in said measuring parts opposite to the measuring test device and parts for arithmetic in said control parts, which correct, as compared with a standard, a difference in a measuring value caused by a fluctuation of a distance between the measuring test device and the detecting part in each measurement, and said measuring parts are constructed as manually transportable.

Here, the " measuring parts are constructed as manually transportable" means that the measurement parts only or the whole analyzer can be transported by hand (carried). The "fluctuation of a distance between the measuring test device and the detecting part" means not only fluctuation from the detecting part but also fluctuation by slant, distortion, etc. at the horizontal or vertical direction.

Briefly, in the present invention, the measuring parts are constructed into capable of transporting manually and fluctuation of distance between the measuring test device and the detecting part, which is caused by the above construction can be corrected comparing with standards. Heretofore, there has never been known or conceived an analyzer wherein measuring parts are constructed into capable of transporting manually and positioned opposite to measuring test device.

The above and other objects and advantages of the invention will become more apparent from the following description.

BRIEF DESCRIPTION OF THE DRAWINGS

Figure 1 is a cross section and a plan showing the roll or sheet of the test device of the present invention.

Figure 2 is a plan showing the roll or sheet of the test device of the present invention.

Figure H is a plan showing other types of the roll or sheet of the test device of the present invention.

Figure 3 is a plan showing the stick of the test device of the present invention.

Figure I is a plan showing other types of the stick of test devices of the present invention.

Figure 4 is a plan showing other types of the test devices of the present invention.

Figure J is a plan showing other types of the test devices of the present invention.

Figure A is a plan showing other types of the stick of test devices of the present invention.

Figure B is a plan showing other types of the stick of test devices of the present invention.

Figure C is a plan showing other types of the stick of test devices of the present invention.

Figure D is a plan showing other types of the stick of test devices of the present invention.

Figure E is a plan showing other types of the test devices of the present invention.

Figure F is a plan showing other types of the test devices of the present invention.

Figure G is a plan showing other types of the test devices of the present invention.

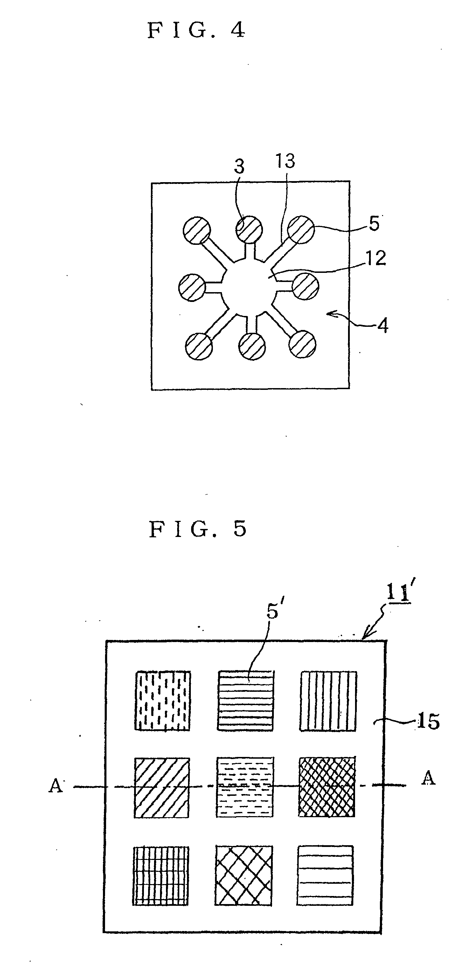

Figure 5 is a plan of test device for measurement of components in a sample originating from a living body of the present invention.

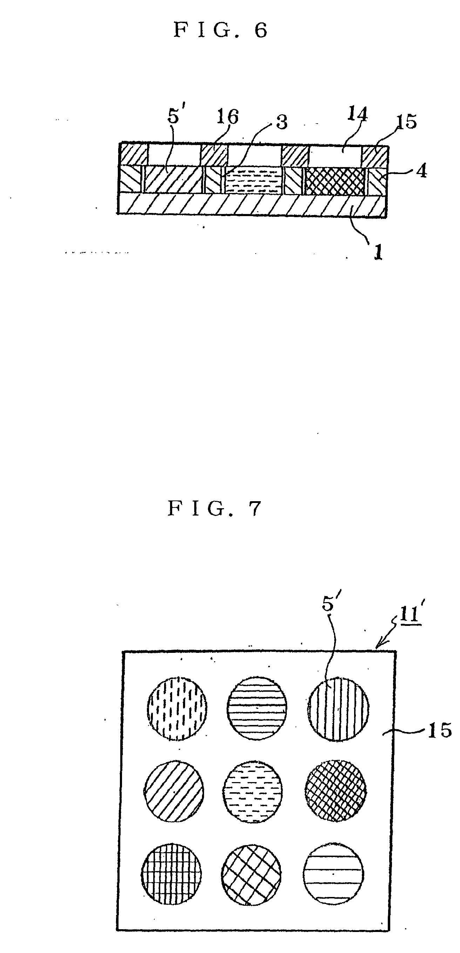

Figure 6 is a cross-section A-A of Figure 5.

Figure 7 is a plan of another example of test device for measurement of components in a sample originating from a living body of the present invention.

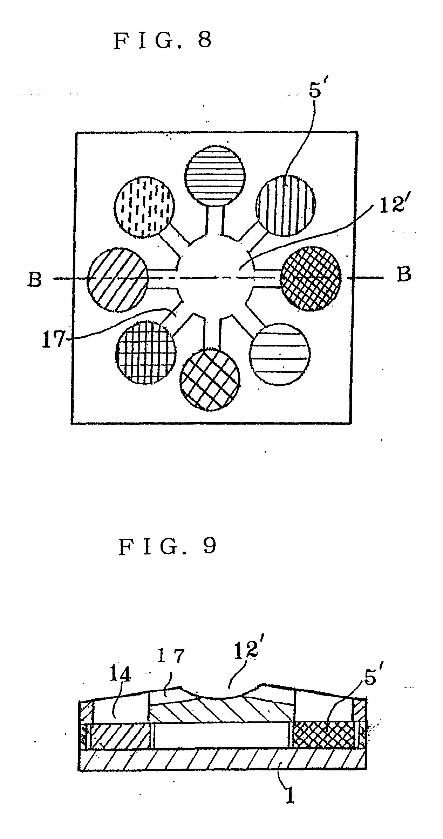

Figure 8 is a plan of still another example of test device for measurement of components in a sample originating from a living body of the present invention.

Figure 9 is a cross-section B-B of Figure 8.

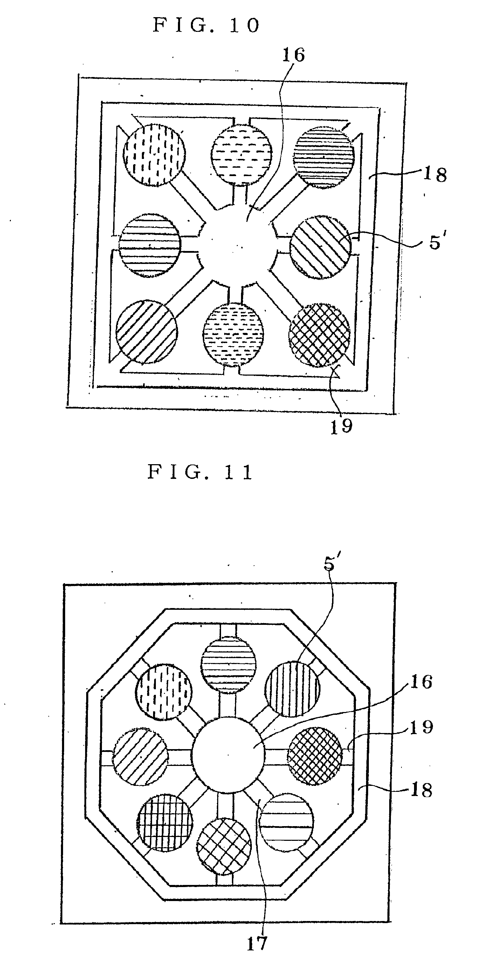

Figure 10 is a plan of another example of test device for measurement of components in a sample originating from a living body of the present invention.

Figure 11 is a plan of still another example of test device for measurement of components in a sample originating from a living body of the present invention.

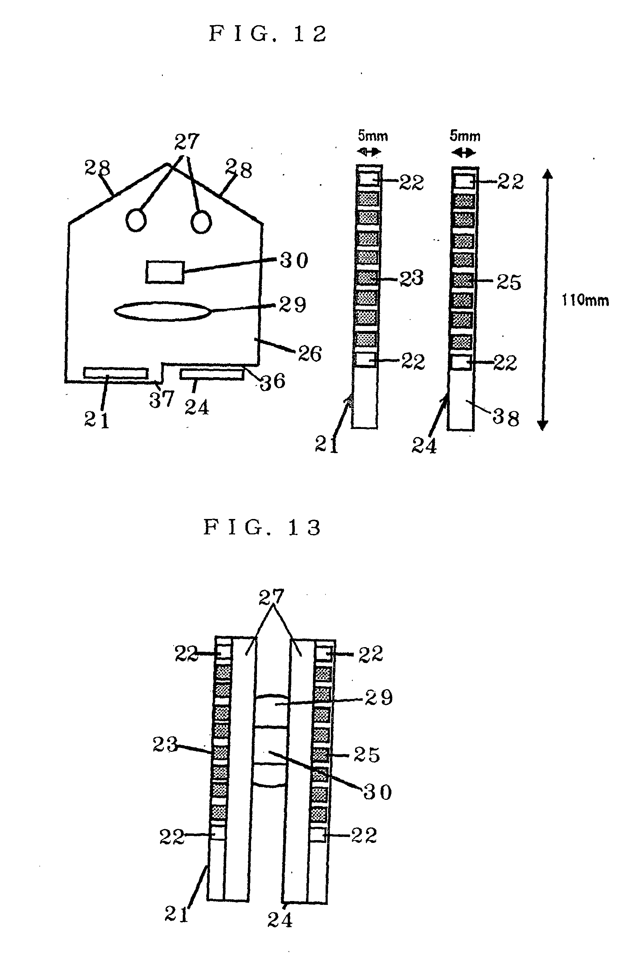

Figure 12 is a cross section showing one example of the measuring parts of the analyzer of the present invention and plan showing one example of the calibrate board and the measuring test device of the present invention.

Figure 13 is a plan showing the inner part of the measuring parts of the analyzer of the present invention.

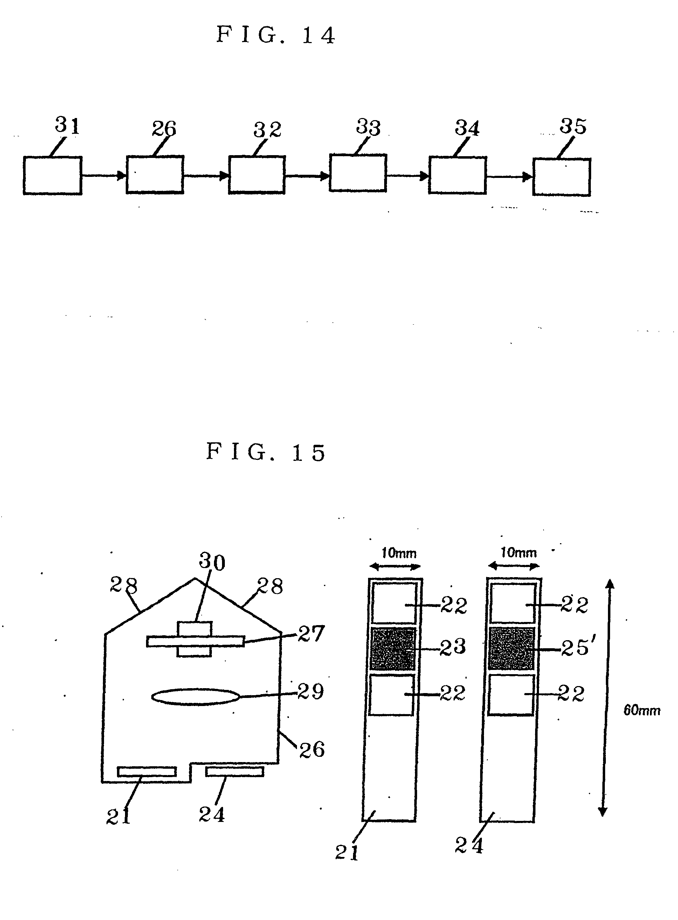

Figure 14 is a block diagram showing one example of the analyzer of the present invention.

Figure 15 is a cross section showing other example of the measuring parts of the analyzer of the present invention and plan showing other example of the calibrate board and the measuring test device of the present invention.

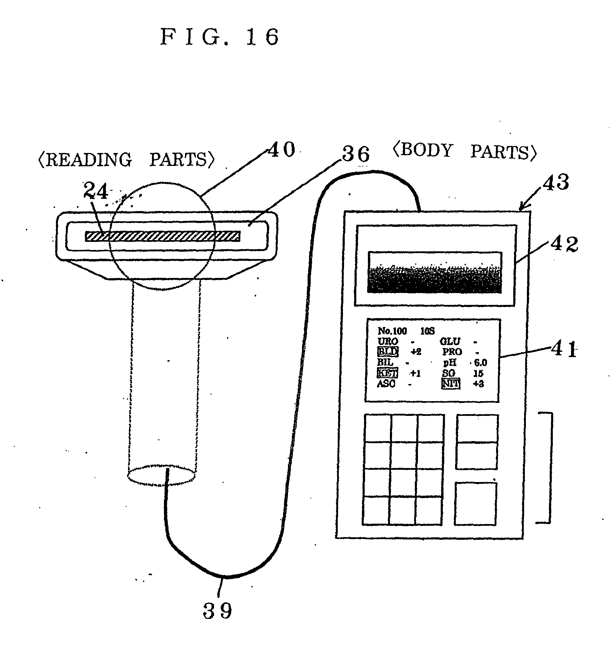

Figure 16 is a perspective view showing one example of the analyzer of the present invention.

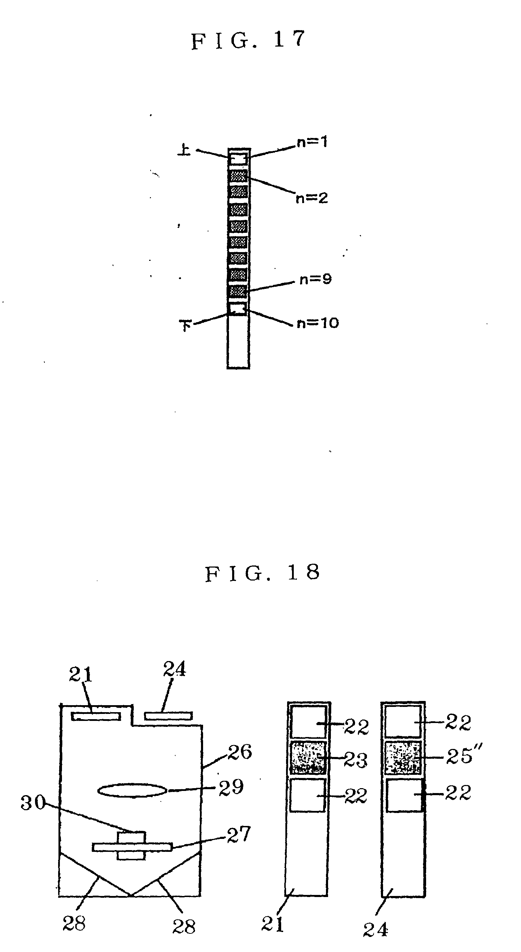

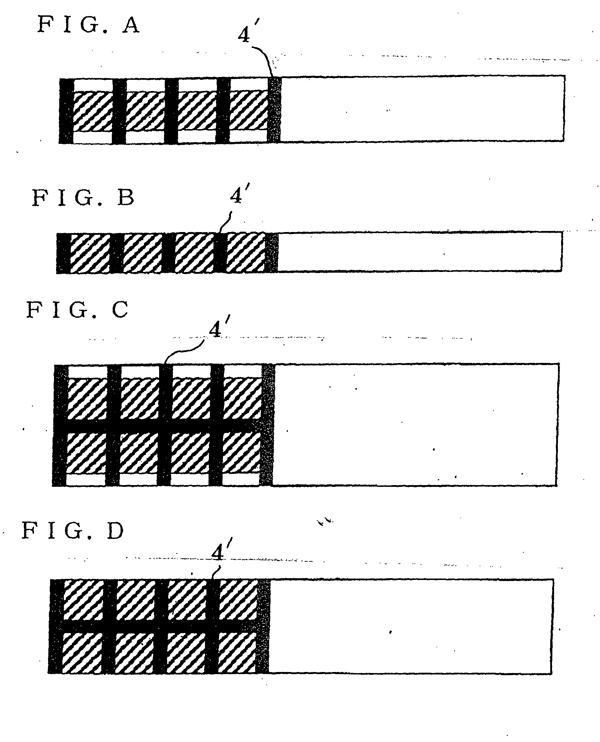

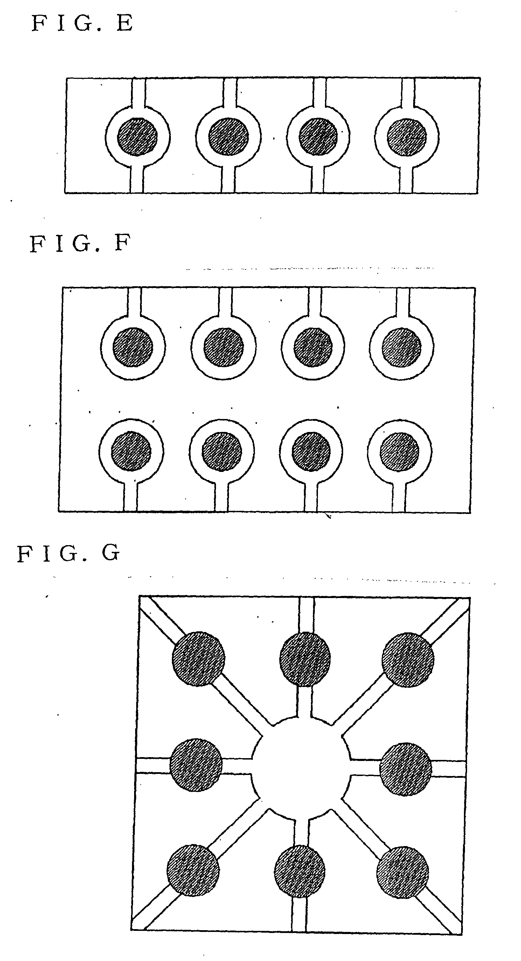

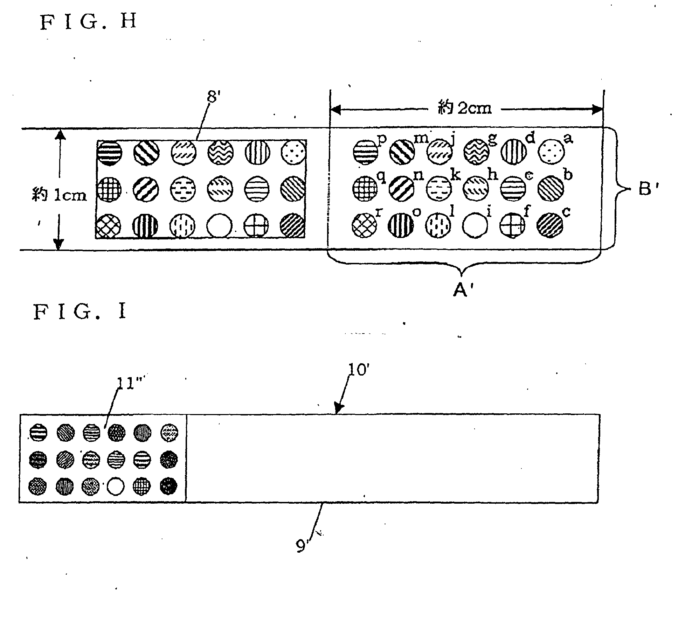

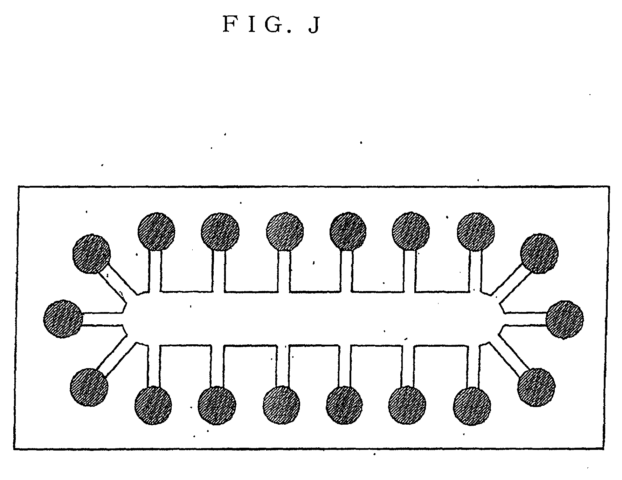

Figure 17 is a plan showing the calibrate board (measuring test device).

Figure 18 is a cross section showing other example of the measuring parts of the analyzer of the present invention.

DETAILED DESCRIPTION OF THE PREFERED EMBODIMENT

In the following, the embodiments of the present invention is explained referring to the Figures.

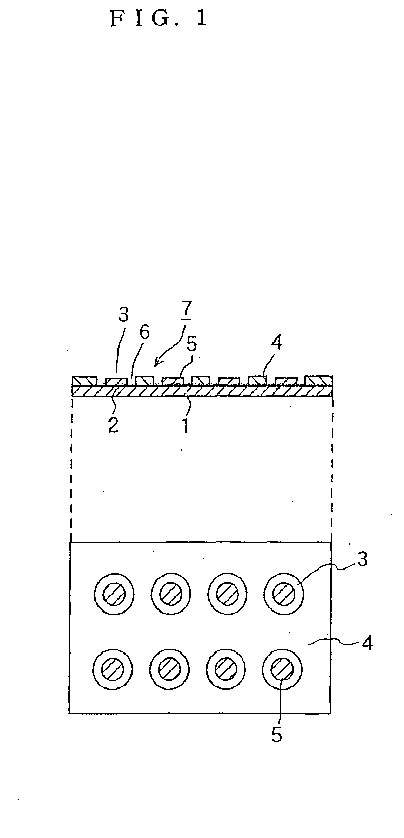

Figure 1 shows an example of the present invention, in which the film (divider wall) 4 formed with a number of circular through-holes forming the concave portions is adhered to the supporting material 1 via the adhesive agent 2 and the circular micro urine test papers or the circular micro test papers for identification of microorganism 5 is adhered to the holes 3.

The urine test paper of the present invention means one obtained by impregnating reagents for measuring various kinds of components in urine (such as glucose, occult blood, pH, proteins, urobilinogen and the like) on an absorbing carrier (such as filter paper, non-woven fabric and the like), followed by drying.

Further, the "Test paper for identification of microorganism" according to the present invention is one in which reagents such as media and various reaction substrate used for identification of various microorganisms (for example, such as enterobacteria and its similar bacterium, staphylococcus and its similar bacterium, dextrose fermentative gram negative bacillus, streptococcus, etc.) contained in samples for example, washing water for semiconductors, a sample originating from a living body including body fluid such as blood, plasma, cereblospinal fluid, gastric juice and bile; urine, feces, sputum, pus, skin and various tissues, tap water, factory waste fluid, food, drink, washing solution of medical instruments, etc. are impregnated in an absorptive carrier (for example, filter paper, non-woven fabrics, etc.) and dried. As various media and reagents used herein, those normally used in this field can be used, and for example, reagents used for identification of microorganism based on the biochemical properties can be mentioned. Also, a microorganism to be identified are not particularly limited ( Clinical Inspection Method Manual, Edition 30, issued August 20, 1993, p. 1063 - 1137; p. 1063 - 1677 "C. Culture Inspection"; p.1078 - 1112 "II. Clinical Bacterium Inspection Of Infectious Disease"; p.1112 - 1121 "III. Acid-fast Bacterium Inspection From Various Materials"; p. 1121 - 1129 "IV. Drug Sensibility Test"; p. 1129 - 1133 "V. Automation Of Bacterium Inspection And Rapid Detection Of Microbial Ethiological Cause"; p. 1133 - 1137 "VI. Manual Of Eumycetes" issued by Kanehara& Co.,Ltd.; descriptions and code books attached to the Entegram for identification of enterobacteria and its similar bacterium, the Staphyogram for identification of staphylococcus and its similar bacterium, the Nonphagram for identification of dextrose fermentative gram negative bacillus other than dextrose non-fermentative and enterobacteria, and the Streptogram for identification of storeoptococcus made of Wako Pure Chemical Industries, Ltd.). Samples used herein include the sample itself described above, or culture solution in which a microorganism separated therefrom is cultivated by media (liquid media) normally used in this field, and so on.

In the present invention, the thickness of the micro test paper 5 is not thicker than the thickness of the film 4. In this way, cross-contamination among the items to be tested which is caused the sample by remained in the area among the adjacent test papers 5 can be prohibited.

Adjustment of the depth of the concave portions to more than the thickness of the micro test paper is for the purpose of preventing peeling-off of the test papers by scrubbing thereof with one another during transportation and preventing degradation of reagents in the test papers by contamination caused by contacting the test devices with one another.

In the above example, the space 6 is formed between the holes 3 and the micro test papers 5. This is not necessarily required, but in this way, the urine test papers 5 can satisfactorily be wetted by a sample such as an urine, a culture solution of a microorganism and the like even when the test papers 5 are formed in considerably small size.

The shapes of the holes 3 and the micro test papers 5 are not specifically limited, and circular or rectangular shapes are preferable from a view point of easy preparation. In this case, by forming one of them into circular (or oval) and the other into rectangular (or polygon), the above mentioned space 6 can easily be formed.

While in the above-described embodiment, the film 4 serves as a divider wall, it is noted that the divider wall may be a side wall in a concave portion, and is not limited particularly.

In the present invention, the area formed by the utmost external line of the series of concave portions necessary for one test per one sample corresponds to the size for wetting the micro urine test papers or the micro test papers for identification of microorganism 5 for all items by one shot dropping of the sample. More concretely, in case of the urine test device, the area is preferably not larger than $2cm^2$, particularly preferably not larger than $1cm^2$, and the length of the horizontal and vertical line is preferably 1.5cm or less, particularly preferably 0.4 to 1.0cm. In case of the test device for identification of microorganism, the area is preferably not larger than $4 cm^2$, particularly preferably not larger than $2 cm^2$, and the length of one horizontal line is preferably 3.0cm or less, particularly preferably 8mm to 2cm, and the length of one vertical line is preferably 1.5cm or less, particularly preferably 4 to 10mm.

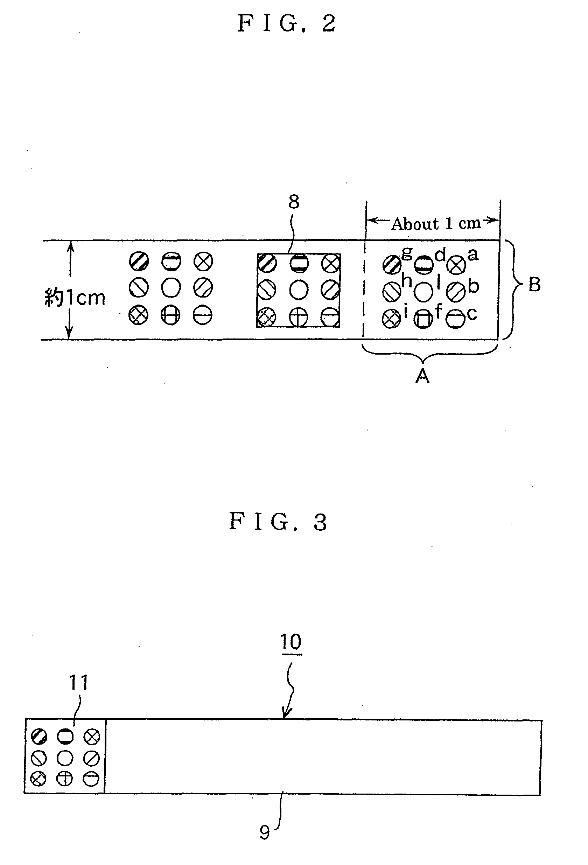

The size of the concave portions (holes) for holding the micro test papers 5 of the present invention is such one as capable of holding a micro test paper. The size is preferably, as shown in Figure 2 and Figure H, so adjusted as 9 or more holes being capable of setting forth per $1 cm^2$ area surrounded by the line 8 and 8' formed by the utmost external side of the concave portions 3 necessary for one test per one sample. By this way, the micro test papers for the whole items can easily and at one time be wet by one shot dropping of the sample.

The size of the micro test papers 5 of the present invention is preferably so adjusted as the diameter or the distance between the two opposite sides of the test papers 5 being 0.5 to 3.0 mm, preferably 0.5 to 2.5 mm. When the size is too small, the measurement sensitivity is decreased, and when too large, it becomes difficult to wet the test papers for the whole items by one shot dropping of the sample.

The length of the horizontal and vertical side of the urine test chip for one test per a sample of the present invention (A and B in Figure 2) is 1.5 cm or less, preferably 4 to 10 mm.

The length (A' in Fig. H) of one horizontal side of a test chip for identification of microorganism for one test according to the present invention is 3 cm or less, preferably, 8 mm to 2 cm, and the length of one vertical side (B' in Fig. H) is 1.5 cm or less, preferably, 4 mm to 10 mm.

Figure 2 and Figure H show an example wherein plural sets of chips for one test per a sample(A×B or A' × B'), and those chips may be separately cut into each chip for a sample for one test.

Fig. 2 shows a multi-items test device (for example, a urine test chip) in which nine micro test papers (a - i) are fitted into nine holes 3 bored in a film of about 1 cm square, and Fig. H shows a multi-items test device (for example, a test chip for identification of microorganism) in which eighteen microtest papers (a - r) are fitted into eighteen holes 3 bored in a film of about 2 cm x 1 cm square. Fig. 4 and J shows another example of a multi-items test according to the present invention. By forming so, sample can be supplied to various micro test papers merely by dropping an sample into a center recessed part.

The numbers of the items formed on one chip for one test per a sample is not less than 4, particularly not less than 8, whereby the test chips can attain satisfactorily the effect of the present invention. Especially, in the test chips for identification of microorganism, the numbers of the items formed on one chips for one test per a sample is not less than 10, particularly 12 to 30.

As in Figure 1, the concave portions 3 are not necessarily covered, but may be covered by a mesh material (net material) or a porous sheet material.

It is preferable to set forth a bar-code or other indication or printing for identification of the item to be tested around the concave portions 3 of the present invention, whereby the item to be tested can easily be recognized.

The film 4 having circular or polygonal holes used in the present invention may be water-permeable or non-water-permeable without specific limitation.

As shown in Figure 4 and Figure J, the concave portions 12 for dropping of a sample are formed in addition to the concave portions 3 for one test per a sample and the concave portions 12 and the concave portions 3 are connected to each other through the ditches 13 having concave shape, whereby the sample dropped in the center part is spreaded to wet easily the test papers for all the items. The above mentioned concave portions 12 and concave portions 3 may directly be connected to each other without forming the ditches 13 of the concave shape acting as a pathway.

The supporting material 1 used in the present invention is preferably one made of a non-water-permeable material such as plastic film, and a material made of a water-permeable material such as paper may be used when the test device is made into a stick like test device as shown in Figure 3 and Figure I.

In Figure 1, a tape whose both sides are sticky or adhesive may be used in place of the plastic film 1 and the adhesive agent 2. Upon using a tape whose both sides are sticky, the peeling paper on the reverse is peeled off after it is make into the situation as in Figure 1 and the resultant is adhered to a stick, whereby a stick like urine test device can easily be prepared.

Figs. A and C show another embodiments of the present invention. A part of a divider wall 4' , that is, the height of a part of a divider wall other than the divider wall partitioning between adjacent test papers is made to be lower than the height of the upper surface of a test paper or substantially equal to the lower surface of a test paper, so that a sample overflown from the concave portions may be discharged. As shown in Figs. B and D, the aforementioned part is not formed, but the side end of a test device may be made substantially equal to the side end of a test paper.

Figs. E, F and G show another embodiments of the present invention.
A ditch is provided externally at the concave portions so that a sample overflown from the concave portions may be discharged.

Since in these embodiments, a stay of a surplus sample into the concave portions caused by surface tension can be prevented, the surplus sample is discharged, and measurement with higher accuracy can be achieved.

Figure 5 and 6 show one embodiment of test device for measuring components in a sample originating from a living body of the present invention, wherein the film 4 formed with a number of rectangular through-holes 3 prepared by die-cut is adhered to the supporting material 1 via the adhesive agent, the rectangular micro test papers 5' for measuring components in a sample originating from a living body are positioned on the holes 3 and the films 15 formed with a number of through-holes 14 are adhered thereon in such a way that the pass-through holes 14 are positioned to the upper places of the micro test papers 5'.

The test device for measurement of components in a sample originating from a living body (except for urine) of the present invention means a test device for measurement of components in a blood sample such as blood, plasma and serum, cereblospinal fluid, saliva, etc. and such device itself has so far widely been used.

The test papers for measuring components in a sample originating from a living body of the present invention is composed of, as in conventional similar kind of test papers, reagent layers capable of reacting with the object components in a sample and developing layers (called also spreading layers or sample-holding layers) made of porous membranes or mesh materials which are laminated in this order on a supporting material. The supporting material for the test papers is not necessarily required, because the supporting material 1 in the test device of the present invention can act also as the supporting material for the test papers.

In the above example, the height of the micro test papers 5' is formed as almost equal to that of the films 4. In this way, since there is no space between the upper divider walls 16 and the micro test papers 5', though there is some space between the films 4 (lower divider walls) and the test papers 5', when the films 15 having through-holes 14 of a size of less than the size of the micro test papers 5' are adhered, the sample can be prevented from impregnating through the sides of the test papers 5' and the test papers can strongly be held even if they are not adhered. When there is a large space between the upper divider walls 16 and the test papers 5' and a sample is impregnated through the sides of the test papers, the sample is not passed through from the upper to the bottom one by one and thus accurate measurement becomes impossible.

It is noted of course that where there is no space (clearance) between the lower divider wall 4 and the test papers 5' so that a sample is not permeated from the side of the test papers, a space may be formed between the upper divider wall 16 and the test papers 5'.

While in the above-described embodiment, a divider wall is formed by the lower divider wall 4 and the upper divider wall 15, and a concave portion is formed by the hole 3 receiving a test paper therein and the through-hole 14, this arrangement is not always necessary.

The capacity of the through-holes (a recessed part) 14 for holding a sample on the test papers 5' is such one as capable of holding a sample for one test, because, in the present invention, the test papers are made into remarkably small and thus impregnation of a sample is not easy, namely a sample is not impregnated immediately after dropping.

The shapes of the holes (the recessed parts) 14 and the micro test papers 5' are not specifically limited. The shape of the holes (the recessed parts) 14 and that of the micro test papers 5' may be same with each other, though not necessarily required.

In the above example, nine nozzles in one distributor are positioned opposite to each of the micro test papers 5', and a sample is, as shown in Figure 5, for instance, capable of dropping at one time on the micro test papers 5' from each of the nine nozzles. The number of the nozzles equipped in the distributor is generally same with that of the micro test papers 5'.

In known test devices for a multi-items test, such contrivance as above was not possible because an amount of a sample to be used was not so large. On the other hand, in the present invention, the test papers can be made into micro size, an amount of a sample for one test can be made into very small and the test papers for all items to be tested can be positioned in a limited area, and therefore the contrivance as above becomes possible.

Namely, in Figure 5, a size of an area formed by the utmost external lines of the concave portions for one test per a sample (and the through-holes 14 in Figure 6) is preferably not larger than 1 $cm^2$ and the length of the horizontal or vertical line is preferably not longer than 2.0 cm, particularly preferably 0.5 to 1.5 cm.

A size of the concave portions 3 (the recessed part 14) to which the micro test papers 5 of the present invention is held is preferably such one that 4 or more, particularly preferably 6 to 9 concave portions are capable of being placed within 1 $cm^2$ of an area formed by the utmost external lines of the concave portions 3 (the recessed parts 14) for one test per a sample. As in example shown in the following Figure 8 to 11, when the concave portion for dropping a sample is set forth on the central part of the concave portions, one concave portion acts as a concave for dropping a sample, and thus the particularly preferable number is 5 to 8.

A size of the micro test papers 5' of the present invention is such that a diameter or a distance between the two opposite sides of the test paper 5' is preferably 0.5 to 3.0 mm, particularly preferably 0.5 to 2.5mm. When it is too small, measurement sensitivity is sometimes reduced, and when it is too large, placement of 6 to 9 concave portions per 1 $cm^2$ area becomes difficult.

A length of a horizontal or vertical line of the test chip (or slide like test device) 11' for one test of the present invention shown in Figure 7 is not longer than 2.0 cm, preferably 10 to 15 mm. The test chip has no frame, but the slide like test device has a frame, and the latter can easily be equipped in a measurement instrument.

A number of the items formed on a chip for one test per a sample is not less than 4, preferably 6 to 9, whereby the effect of the present invention can satisfactorily be realized.

Bar-codes for identification of the items to be tested or indications or printing for this purpose are preferably set forth around the concave portions 3 (concave portions 14) of the present invention.

The film 4, 15 having circular or polygonal holes used in the present invention is not specifically limited and may be water-permeable or non-water-permeable, including for example non-water-permeable one such as a synthetic polymer including polyethylene, polystyrene, polyester, polyurethane, etc. and water-permeable one such as cardboard, among which non-water-permeable one is preferable.

The supporting material 1 used in the present invention is preferably non-water-permeable one such as a plastic film made of a synthetic polymer including polyethylene, polystyrene, polyester, polyurethane, etc. but may be water-permeable one such as paper. When the measurement is conducted from the downward direction (measurement of reflection light from the reverse of the test devices), use is preferably made of a transparent plastic material.

As the micro test papers are used in the present invention, a sample such as blood etc. is difficult in impregnation into a reagent layer of the test papers when a small amount of a sample is dropped. Thus, setting forth for means for easily impregnating a sample into the micro test papers is desirable.

Preferable means for easily impregnating a sample into the test papers includes subjecting a surface developing layer of the micro test papers to highly hydrophilic treatment. The hydrophilic treatment is preferably coating an upper surface of the test papers with a surfactant so long as it has no bad influence upon the measurement and other conventional hydrophilic treatments.

Further, preferably, the supporting material (a thin sheet) 1 to which the bottom surface (reverse) of the micro test papers is contacted is equipped with pores or made into mesh so as to deflate air to outer atmosphere, whereby a sample is easily impregnated.

It is preferable to form pores or make into mesh as mentioned above and set forth means to press from the upper surface (obverse) of the micro test papers or set forth means for reducing pressure from the bottom surface (reverse) of the micro test papers so that impregnation of a sample on the surface becomes easy. Such means for pressing or reducing pressure may be incorporated into means for detection.

Means for pressing includes a sucking disk and the like equipped on the test papers, and means for reducing pressure includes a sucking disk and the like equipped on the bottom surface (reverse) of the test papers.

The test device for measuring components in a sample originating from a living body other than an urine of the present invention can be constructed, as in known similar test devices, as capable of measuring reflection lights from the obverse of the test devices and/or those from the reverse of the test devices. In a case of measuring reflection lights from the reverse, it is preferable to use a transparent sheet as the supporting material 1 as in known devices or to construct the through-holes. It is also possible to conduct the measurement by an electrode method, and in this case, use is made of not only a sample but also a reference solution.

Figure 8 and 9 show other examples of the present invention wherein the concave portions for dropping of a sample 12' is fromed in addition to the concave portions for one test 14 and the both concave portions are connected through concave ditches 17 as pass-ways and thus a sample dropped on the concave portion for dropping of a sample 12' placed on the central part is spreaded to wet easily the test papers 5' for all of the items to be tested. The concave portion for dropping of a sample 12' and the concave portions for one test 14 may directly be connected with each other without forming the concave ditches 17.

In the above examples, the bottom of the concave portion for dropping of a sample 12' is positioned at higher level than that of the concave portions for one test per a sample 14 and the ditches 17 for connecting the both concave portions are formed in slanting downwardly. In this way, a sample dropped on the concave portion for dropping of a sample 12' is easily flown on the test papers 5'. The concave ditches 17 are not necessarily formed in slant downwardly, so long as they are formed in such a way as capable of transporting a sample from the concave portion for dropping of a sample 12' to the concave portions (the recessed parts) 14. As shown in Figure 9, the concave ditches 17 acting as a path-way for a sample is formed in such a way as capable of introducing a sample downwardly on the test papers 5' and preventing a sample from introducing from the side of the test papers 5'.

In this example, the position of the bottom of the concave portion for dropping of a sample 12' is positioned to the same level with that of the concave portions for all items 3 (the recessed part 14), and thus a predetermined amount of a sample can be introduced into the concave portions for all items 3 (the recessed part 14) by pouring a sample up to this level.

Figure 10 shows still another example of the present invention, wherein ditches 18 for holding an excess amount of a sample is formed outside of the concave portions for one test 3 (or the recessed part 14), and an excess amount of a sample over-flown from said concave portions 3 (or the recessed part 14) is contained in the rectangular ditches 18 from the concave pass-ways 19. In place of the ditches 18, mere pores (not connected with one another) may be formed.

In the above example, an excess amount of a sample is over-flown, and thus a predetermined amount of a sample can easily be flown into the concave portions 3 (or the recessed part 14).

Figure 11 shows still further example of the present invention, wherein distances between the concave portion for dropping of a sample 12' and each of the concave portions in which the test paperes 5' for each of the items to be tested are nearly the same with one another. In this example, each of the concave portions for one test per a sample is formed in ring around the concave portion for dropping of a sample 12'. In this way, a predetermined amount of a sample can be introduced in each of the concave portions for one test per a sample more accurately.

While in the embodiments shown in Figs. 5 to 11,the test device has been explained as a test device for measuring a living-body component, it is noted of course that can be also used as a urine test device or a microorganism test device.

Then, a method for preparation of the test device of the present invention which is composed as Figure 1 to 3 is explained below.

As shown in Figure 2, many of the set composed of the number 3 x 3 of the circular holes 3 having a diameter of 2mm are formed by die-cut on a polymer sheet 4 having about 10 mm width and about 0.5 mm thick.

Then, thus prepared polymer sheet 4 is adhered to a tape whose both sides are sticky, and the 9 kinds of the test papers a to i which are prepared by die-cut into circular shape of 1.8 mm diameter are adhered to each of the holes 3.

In this process, the tape whose both sides are sticky is unrolled from a roll while the releasing paper is peeled off, and the polymer sheet 4 having the holes 3 which is unrolled downward is adhered to the unrolled tape by a pressing roll, whereby a continuous preparation of the test device can be conducted.

Upon adhering the 9 kinds of test papers a to i, at first plural numbers of the test papers a are adhered at the lengthwise direction, and then the same processes are repeated one by one also on other test papers b to i.

Adhesion of plural numbers of test papers a to i at the lengthwise direction is preferably conducted as follow.

(1) The micro test papers having the desired size and shape, which are prepared by die-cut with the use of a cutter or a die-cut tool (so-called Thomson blade) composed of a wooden plate having a blade (such as a razor blade), are aspirated into a column under reduced pressure to hold the papers therein, and the column is transported to the holes placed on the desired positions of the adhered sheet or to the desired positions of the sheet which correspond to the holes after adhesion and then the column is adjusted to be opposite to the holes or positions, followed by placing and adhering to the above mentioned thin sheet like plate as supporting material 1 (a first sheet material) by increasing the inner pressure to normal to elevated pressure.

(2) The above mentioned micro test papers having the desired size and shape which are prepared by die-cut after the same manner as above are pierced by a tool having one or plural numbers of needles to hold them in the needles, and then the tool is transported to the holes placed on the desired positions of the adhered sheet or to the desired positions of the sheet which correspond to the holes after adhesion and then the column is adjusted to be opposite to the holes or positions, followed by taking out of the needles and placing and adhering to the above mentioned thin sheet like plate.

(3) The micro test papers a are prepared by die-cut with the use of plural numbers of columns equipped at the edge with blades having the same shape as the object test papers, and the resulting test papers a are held in the column by keeping the column under reduced pressure, and then the column is transported to the positions opposite to the holes 3 corresponding to each item to be tested on the polymer sheet 4, followed by adhering the die-cut urine test papers a to the holes 3 by increasing the inner pressure of the column to normal or slightly elevated pressure.

After anyone of the above mentioned process (1) to (3), the same processes anyone of as the above (1) to (3) are conducted also on the test papers b to i to adhere them to the holes 3, thereby there is obtained the roll or sheet of the urine test device 7 wherein the test device (chips) 11 of the present invention as shown in Figure 1 and Figure 2 are continuously equipped. Then, if necessary, a net material such as a nylon mesh is adhered to the surface. The resultant can be used as it is for an automatic analysis.

Additionally, according to the purpose, the device is cut into a square material having about 1 cm x about 1 cm size for one test per a sample as shown in Figure 2, whereby the test chip 11 of the present invention is obtained.

As shown in Figure 3, the stick like test device 10 can be prepared by peeling off the releasing paper on the reverse of the test chip 11 and adhering the resultant to another polymer sheet (stick) 9.

Instead of the above examples wherein the polymer sheet is adhered to the tape whose both sides are sticky and then the micro test papers are adhered thereto, the micro test papers having the desired size and shape may be adhered to the tape, followed by adhering the polymer sheet 4 thereto. In this case, the positions to which the micro test papers are adhered are coincident to the positions of the holes 3 of the polymer sheet 4 to be adhered.

Further, instead of the above (1) to (3) wherein the columns holding the micro test papers are transported, the sheet like plates (the tape or the adhered sheet) may be transported so long as the columns can be adjusted to be opposite to the holes of the polymer sheet.

Almost constant amount of a sample can be supplied to the concave portions 3 of the present invention, and therefore the test device of the present invention can be used not only for qualitative but also for half-quantitative analysis.

Then a method for preparation of the test device for measurement of components in a sample originating from a living body other than an urine of the present invention which is composed as Figure 5 to 7 is explained below.

As shown in Figure 5 and 6, tapes whose both sides are sticky are adhered to the both surfaces of the films 4, the number of 3 x 3 of rectangular holes 3 are formed by die-cut, releasing papers on one surface are peeled off, the resultants are adhered to the film 1 acting as the supporting material 1, and the micro test papers 5' prepared by die-cut in predetermined size are inserted to the other surface of the holes 3. Then releasing papers on the other surface are peeled off, and the films 15 wherein the number 3 x 3 of holes having almost the same or smaller size compared with the holes 3 in the film 4 are formed by die-cut are adhered to said films 4 in such a way that the holes 3 and the holes 14 are coincided with one another, whereby there are obtained the chips 11' (or slide like test devices) of the present invention as shown in Figure 5 and 6.

The tape whose both sides are sticky are adhered to the both surfaces of long rectangular polymer sheets, plural sets of holes composed of the number of 3 x 3 of the holes 3 are formed by die-cut, long rectangular films 1, 15 are adhered as mentioned above and the test papers are placed on the holes 3, whereby roll-like or sheet-like test devices having said chips 11' formed continuously can be obtained. The devices themselves can be used as devices for biochemical automatic analyzers, and alternatively the test chips 11' of the present invention can be obtained by cutting them into one for one test per a sample shown in Figure 5.

In the above example, the films 15 are laminated on the films 4, but only the films 4 may be enough when a thickness of the films 4 is larger than that of the test papers and it is enough to form a concave portions (a recessed part) of such a depth as capable of holding a sample for one test thereon after adhesion of said micro test papers.

The micro test papers 5' may be only placed on without adhesion or adhered to the films 1, but in a case of the test devices for measurement of reflection lights from the reverse of the test devices wherein the measurement is conducted from the reverse of the test devices, no adhesive agent can be used and thus they are to be only placed on the films. Even in such a case as above, the test papers can be strongly held as if they are adhered, when the holes 14 in the films 15 are smaller in size than the holes 3 in the films 4 whereby the thickness of the holes on the films 4 can be almost the same with that of the test papers.

In the above example, after the films 4 are adhered to the tapes whose both sides are sticky, the micro test papers are adhered, and it may be admissible that the micro test papers having predetermined size and shape are adhered to the tapes whose both sides are sticky and then the films 4 having the holes 3 prepared by die-cut from the upper part thereof are adhered. In this case, the positions to which the micro test papers 5' are adhered are, needless to say, coincided to the positions of the holes 3 in the films 4 which is adhered later on.

In a case of preparation of the test papers for measuring reflection lights from the reverse of the test devices wherein the measurement of light is conducted from the reverse of the test devices, the tapes whose both sides are sticky are adhered to the both surfaces of thin sheet like plate made of a transparent material, many through-holes for positioning the micro test papers are formed, releasing papers on one surface are peeled off, sheet materials having many through-holes for deflation of air are adhered thereto, said micro test papers are adhered to or tightly placed on the holes or the places corresponding to the holes of said thin sheet like plate, another sheet like substances having many holes are, after releasing papers on the other surface of said thin sheet like plate are peeled off, adhered thereto in such a way that the holes of said thin sheet like plate and those of the second sheet materials are overlapped with each other.

Placing or adhering the micro test papers can be conducted, for instance, the same as above mentioned processes (1) to (3) of adhesion of 9 kinds of the test papers.

The test device of the present invention which is composed as Figure 4, A to J can be prepared by the same processes as the above examples.

The measurement by the test device for measurement of components in a sample originating from a living body of the present invention can be conducted by convention methods including methods for measuring reflection lights from the obverse of the test devices, method for measuring reflection lights from the reverse of the test devices and electrode methods.

The micro test papers of the present invention is remarkably small in size, and thus the test devices on which the papers are adhered are used not for visual observation of the test result but for observation by an analyzer.

The test device of the present invention is in such a size that all test papers for all items to be tested can be wet by one shot dropping of a sample and further the papers for each items to be tested are placed collectively on a very limited area, and therefore the reflection lights after irradiation with white light or light of a specific wave length from a small light source can be measured at one time (with no transportation period) without mechanically transporting the test devices or moving the irradiation and measuring points. The "at one time" does not means "strictly at the same time" but includes also "measuring each items one by one with almost no moving time of the test devices or the light source".

In other words, in the present invention, the concave portions for the number of one test sample are provided within the range capable of measuring a detecting part or test devices without moving every item.

Therefore, in case of small size instrument for measurement of small numbers of samples, the moving part of the analyzer can be omitted, and in case of large size instrument for measurement of large numbers of samples, the analyzer can be made into compact by making the test device into chips and reducing the moving parts.

In the present invention, many items can be measured in one shot dropping of a sample, and further the measurements can be conducted at one time as mentioned above, and therefore remarkably high speed tests can be attained which has so far never be realized.

The method for the measurement of the test device by the present invention can be conducted theoretically by the use of a UV spectrometer or a fluorescent spectrometer, and generally white light or a light having a specific wave length is irradiated and then the reflection light is measured. For this measurement of reflection light, use is made of a visible ray spectrometer, CCD sensor, etc.

In the present invention, the test papers for each of items can collectively be adhered within the pre-determined area because the micro test papers are used, and therefore in case of measuring many items, dipping can easily be conducted in a dipping method and the measurements can be conducted only with one shot dropping in a dropping method.

Still further, in conventional dipping methods, there has been such a fear that a part of ingredients (reagents etc.) contained in test papers is leaked to contaminate a sample whereby other measurements are influenced. In the present invention, on the other hand, such an influence as mentioned above can remarkably be reduced because the size of the test papers is very small.

In known multi-items test papers, dropping of a sample can be conducted in one shot only for one item to be tested, and thus transportation of a distributor or test devices is required for multi-items measurement. On the other hand, in the present invention, dropping of a sample can be conducted in one shot for all the items to be tested, and thus transportation of a distributor or test devices is not necessary at all, which results in making the instrument compact, reducing time for transportation and increasing measurement speed and further reduction of electric power to be consumed.

In conventional test devices, distance among each test paper is relatively far, and it is necessary, upon detection, to mechanical transport test devices or detecting part for the desired positioning. In the present invention, on the other hand, it is not necessary any more to transport test papers or detecting parts, which results in making handling simple, making the instruments compact and increasing measurement speed and further reduction of electric power to be consumed.

Therefore, the analyzer can be made into remarkably compact by making the test devices into chip type and reduction of moving parts.

Further, in the present invention, a size of the test papers used can be minimized to a large extent as compared with known test devices and the necessary items can be measured collectively in a limited area, and therefore an amount of the expensive test papers can be reduced to a large extent which makes it possible to supply the test devices (chips) of the present invention at considerably low cost. Further, in this way, an amount of wasted materials can also be reduced, which can make great contribution to the protection of natural resources and environments.

In the following, embodiments of the analyzer for detecting components of a sample in a measuring test device of the present invention are explained.

As the measuring test devices usable in the analyzer of the present invention, there have been mentioned urine test devices, immunochromatographic test devices, measuring test devices for detecting biochemical components (such as glucide, lipid, protein, and the like), dry chemistry films, a microorganism test device, etc., among which use is preferably made of urine test devices or a microorganism test devices.

As the light source, use is made of LED, fluorescent lump, tungsten lump, etc., among which LED is preferably used because it can make the analyzer minimized and prevent heat generation and small size fluorescent lump is also preferably used because it makes the analyzer minimized.

The left side of Figure 12 is a rough cross section showing one example of the measuring parts (detecting modules) 26 of the present invention, wherein the bottom end of the module 26 is constructed in stairs and the concave part of said stairs constitutes the reading parts 36 for the measuring test device. The reading parts 36 for the measuring a test device may be constructed into concave or into flat instead of stairs.

The measuring test device 24 is positioned near to the reading parts 36 for the measuring test device, and the calibrate board 21 is held in the projecting part 37 of the bottom edge of the detecting module 26.

The measuring test device 24 is composed of, as shown in Figure 12, the test papers 25 (8 numbers) for each items to be tested and the white standard sheets 22,22 which are positioned so as to put the 8 numbers of the test papers 25 between them from upper and lower sides, the both being held on a slender plastic stick 38.

The calibrate board 21 is, as shown in Figure 12, composed of the colored standard sheets 23 (8 numbers) having the colors developed from the test papers 25 on the items to be tested (colors often developed) and the white standard sheets 22,22, and the test papers 25 and the white standard sheets are positioned in such a way as respective items being opposite to each other.

When the plastic stick 38 is constructed as acting also as the white standard sheets, the white standard sheets 22 are not necessarily set forth. It is enough to set forth white standard parts. However, for more accurate measurement, it is preferable to conduct detection in a definite picture, and thus it is preferable, as shown in Figure 12, to adhere the white standard sheets 22,22 to predetermined positions on the plastic stick 38.

The white standard sheets 22 are preferably set forth in plural numbers in such a way as putting the test papers between them, particularly preferably in such a way as setting forth at the outside positions of the both edges of the test papers 25, as shown in Figure 12. The correction can be attained even by setting forth the white standard sheets only at one position and measuring reflection lights from two positions in the white standard sheets, but for more accurate measurement, two or more of the white standard sheets are set forth and the white standard sheets are detected as pictures (photographic) images.

As the white standard sheets 22, use is preferably made of those made of such a material as repellent completely against the sample including an urine or a blood sample such as blood, serum and plasma, a culture solution of microorganism, and the like or made of a suitable material coated with a substance completely repellent against the sample, in other words, those made of such a material as causing no change in the reflection lights before and after immersion in the sample, because adhesion of the sample causes apparently large changes in the reflection lights from the white standard sheets 22 on the calibrate board among each of the items to be tested. The white standard sheets 22 in the measuring test devices and those in the calibrate board are made of the same material so that the same reflection signals are made.

As shown in Figure 12 and Figure 13, the measuring optical system comprising the lens system 29, the detecting part (instrument) 30 and the light source 27,27 are set forth upwards the calibrate board 21 and the measuring test device 24. As the lens system, use may be made of plural numbers of lenses and the lens may be equipped with an iris. The lens is, however, not necessarily required.

The lens system 29 takes a role of image formation of the pictures of the white standard sheets 22 and the colored standard sheets 23 on the calibrate board 21 and the white standard sheets 22 and the test papers 25 on the measuring test device 24 on the detection scene of the detecting part, and the detecting part 30 has a function of measuring separately into R, G and B the doses of the reflection lights from the white standard sheets 22 and the colored standard sheets 23 on the calibrate board 21 and from the white standard sheets 22 and the measuring test sheets on the measuring test device 24.

As the detecting part 30, any sensor having photo-receptor elements can be used, and photo-cells, image sensors and CCD sensors are mentioned as examples.

Photo-cells can correct only brightness (black-and-white), and it cannot differentiate normal color development from abnormal color development when the latter is caused, and therefore, color image sensors or color CCD sensors which can correct also color information (hue, chroma, brightness, etc.) are preferably used. Further, for obtaining accurate data, CCD sensors, particularly color CCD sensors, are preferably used.

The spreading board 28 is equipped on the upper ceiling part of the light source 27.
By this spreading board 28, lights from the light source can be irradiated on the calibrate board 21 and the measuring test device 24 homogeneously without unevenness, and thus accurate measurement can be conducted.

Figure 14 is a block figure showing one example of the analyzers of the present invention, which is composed of the stabilized electric power source 31 for stabilizing the power of the light source of the detecting module 26 and the control parts (module body) comprising the signal output (·amplification)·A/D conversion circuit 32, the memory (including memory for memorizing signals and memory for memorizing standard values) 33, the operation parts 34 and the data-output parts 35.

The signal output (• amplification) • A/D conversion circuit 32 takes a role of converting the analogue values corresponding to the dose of light measured to the digital values and putting them into the memory part in the memory for memorizing signals 33, the memory part being previously determined corresponding to the kinds of the signals. The amplification in the parenthesis means that it may or may not be possessed.

The memory for memorizing the standard values have the function of measuring the reflection lights from the calibrate board 21 carrying the standard sheets 23 having the colors developed by the test papers for the items to be measured and the white standard sheets 22 from a predetermined distance from said detecting part 30 and memorizing the resultant separately into R, G and B by each of the standard sheets.

The operation parts 34 are for operating the corrections on the measuring optical system and on the measuring objects with the use of the data of said memories for memorizing the signals and for memorizing the standard values and then determining ranking on each of the items to be measured.

Thus obtained result of the ranking can be indicated on the liquid crystal display (LCD) on the data-output parts 35 and printed out.

Figure 15 shows another example of the present invention wherein it is applied to micro test devices.

Namely, the micro multi-items test devices 25' in the measuring test device 24 are set forth in such an area that the micro test devices for all of the items to be tested for one test per a sample can be wet by one dropping of the sample, and the size of the micro test papers is such as the diameter or distance between the two opposite sides of the micro test papers being generally 0.5 to 3.0 mm, preferably 0.5 to 2.5mm.

In the above example, 3 numbers of the multi-items test papers at horizontal direction and 3 numbers thereof at the vertical direction, totally 9 numbers thereof, are fixed on the multi-items test device 25' of 1 cm$^2$ area (figure omitted).

In this example, the construction is made similarly to the example shown in Figure 12 and Figure 13, except for using a ring light source 27 as the light source 27. It is preferable to construct the light source having the same shape as the shape of the object to be measured because homogeneous irradiation can be conducted. In the above micro test paper, the outer shape of the construction is almost square, and thus the light source is constructed in the ring shape. In the example shown in Figure 12, use is made of a stick like test device arranged in straight lines, and thus the right column light source is used.

The calibrate board 21 is constructed in such a way as putting the colored standard sheets 23 having the colored standard sheets between the white standard sheets 22, 22 from the upper and the bottom directions. The measuring test device 24 is constructed in such a way as putting the multi-items test device 25' between the white standard sheets 22, 22 from the upper and the bottom directions. Also in the colored standard sheets, as in the micro test papers equipped in the multi-items test device 25', 9 numbers of the colored standard sheets (figure is omitted) are held on the same positions as in the micro test papers. For further accurate measurement, it is preferable to put further the multi-items test device 25' between the white standard sheets 22,22 from the left and the right directions. In this way, the slant at the left and right directions of the test device 25' can more accurately be corrected.

Figure 16 shows a concrete example of the present invention, wherein the measuring parts (reading parts) is constructed in bar-code-read type and connected to the control parts (the body parts) 43 by lead wire 39. The reading parts 36 is formulated in concave, and it is constructed in such a way as capable of measuring only by facing it on the measuring test device 24 on the cup 40 from the upper direction . In Figure 16, 41 shows the display parts, and 42 shows a printer. Data exchange between the measuring parts and the control parts may be conducted by using radio transmission means such as infrared transmission means instead of connection by the lead wire 39.

In the present invention, measurement can be conducted even when the reading parts of the test device and the test device for the measurement may be distant with each other, but the distance is not unlimited. So long as certain dose of light usable as data can be obtained from the white standard sheets and the test papers formed oppositely to the measuring test device and it can be imaged as a certain kind of shape on the detecting part, the measurement can be achieved. It is preferable to formulate in such a way that when the measurement becomes possible as mentioned above, beep sound generated on reading by a bar-code reader or other sound is generated or an indication is appeared on the display parts 41.

In the following is given explanation of operations in the operation parts (parts for arithmetic) 34. In the operation parts, there are conducted (A) corrections on the measuring optical system, (B) corrections on the objects to be measured, (C) operations of reflection rates of each item to be measured and (D) determination of ranking on each item to be measured. The corrections (A) include corrections on the white standard sheets (correction 1) and corrections on the colored standard sheets (correction 2), and the corrections (B) include corrections on the white standard sheets (correction 3) and corrections on the test papers (correction 4). In the following is given explanation of each of them.

The reflection lights from the calibrate board 21 having the colored standard sheets 23 which are colored with the same color as developed by the test papers 25 for the item to be measured and the white standard sheets 22 are measured at a predetermined position distant from said detecting part (using color-CCD-sensor) 30 and memorized in said standard memory as the standard value. In this case, the memorization is conducted in such a way that the signals of the reflection lights from the upper white standard sheets of the calibrate board and those from the bottom white standard sheets are adjusted to the same with each other.

(A) corrections on the measuring optical system: fluctuation by the measuring optical system is corrected.

(correction 1) correction on the white standard sheets: the measured values on each of the standard sheets on the upper and the bottom positions on the calibrate board are corrected. Correction coefficients on the (upper) standard sheet and the (bottom) standard sheet are obtained according to the following equations. In the equations, the (upper) correction coefficient 1 shows the correction coefficient on the (upper) standard sheet and the (bottom) correction coefficient 2 shows the correction coefficient on the (bottom) standard sheet. Those corrections are corrections on fluctuations of power of the light source and fluctuations on sensitivity of detection of the detection instrument.

(upper) correction coefficient 1 = (upper) standard value on the standard sheet on calibrate board/(upper) measured value on the standard sheet on calibrate board

(bottom) correction coefficient 1 = (bottom) standard value on the standard sheet on calibrate board/(bottom) measured value on the standard sheet on calibrate board

In this example, the standard values of the upper and the bottom standard sheets are adjusted to be the same with each other.

(correction 2) correction on the colored standard sheets: each of the measured values on R, G and B on each of the colored standard sheets (n=2 to n= 9 in Figure 17) on the calibrate board are corrected. According to the following equations, each of the correction coefficients on R, G and B on each of the colored standard sheets 2 to 9 are obtained. In the equations, n is 2 to 9, m is R, G or B. Those corrections are corrections of fluctuations of luminescent waves from the light source and fluctuations of detected wave characteristics from the detecting part.

correction 2 nm coefficient = nm standard value of the colored standard sheets on the calibrate board/(measured nm value of colored standard sheets on the calibrate board x A)

in which A means the following;

in case of correction 1 (upper) coefficient > correction 1 (bottom) coefficient

$$A = \text{correction 1 (bottom) coefficient} + (\text{correction 1 (upper) coefficient} -$$

$$\text{correction 1 (bottom) coefficient}) \times (10 - n/10\text{-}1)$$

in case of correction 1 (upper) coefficient $\leqq$ correction 1 (bottom) coefficient

$$A = \text{correction 1 (upper) coefficient} + (\text{correction 1 (bottom) coefficient} -$$

$$\text{correction 1 (upper) coefficient}) \times (n\text{-}1/10\text{-}1)$$

(B) corrections on the objects to be measured: corrections are made on the white standard sheets and on the test papers.

(correction 3) corrections on the white standard sheets: the measured values on the upper and bottom white standard sheets on the measuring test device are corrected. According to the following equations, correction coefficients on the (upper) white standard sheets and the (bottom) white standard sheets are obtained. In the equations, the correction 3 (upper) coefficient shows the correction coefficient on the (upper) white standard sheet, and the correction 3 (bottom) coefficient shows the correction coefficient on the (bottom) white standard sheet. Those corrections are corrections on the vertical distance and the horizontal twist between the test device and the measuring parts.

correction 3 (upper) coefficient = the standard value on the (upper) white standard sheets/(the measured value on the (upper) white standard sheet on the test device x correction 1 (upper ) coefficient)

correction 3 (bottom) coefficient = (bottom) the standard value on the (bottom) white standard sheet/(the measured value on the (bottom) white standard sheet on the test device x correction 1 (bottom) coefficient)

The (upper) standard value on the white standard sheet on the calibrate board and one on the (bottom) white standard sheet on the calibrate board are each used as the standard value on the (upper) white standard sheet and the standard value on the (bottom) white standard sheet respectively.

(correction 4) corrections on the test papers: each of the measured values on R, G and B on each of the test papers on the measuring test device (n=2 to 9 in Figure 17) are corrected. According to the following equations, each of the correction coefficients on each of R, G and B on the test papers n=2 to 9. In the equations, n and m have the same meaning as above.

nm correction value on the test papers on the measuring test device = the nm measured values on the test papers on the measuring test device x correction 2nm coefficient x B

in which B means the following;

in case of correction 3 (upper) coefficient $\geqq$ correction 3 (bottom) coefficient

$$B = \text{correction 3 (bottom) coefficient} + (\text{correction 3 (upper) coefficient} -$$

$$\text{correction 3 (bottom) coefficient}) \times (10\text{-}n/10\text{-}1)$$

in case of correction 3 (upper) coefficient $\leqq$ correction 3 (bottom) coefficient

$$B = \text{correction 3 (upper) coefficient} + (\text{correction 3 (bottom) coefficient} -$$

$$\text{correction 3 (upper) coefficient} \times (n\text{-}1/10\text{-}1)$$

in which n and m have the same meaning as above.

(C) operations of reflection rates on each items to be measured

Reflection rates on each items to be measured are operated according to the following equation with the use of the above obtained corrected nm values on the test papers on the measuring test device and the standard value on the (upper) white standard sheet on the calibrate board.

nm reflection rate on of the test papers on the measuring test device = (corrected value on the test papers on the measuring test device/standard value on the (upper) white standard sheets on the calibrate board) x 100

in which n and m have the same meaning as above.

(D) ranking determination on each items to be measured

For instance, determination is conducted as to whether reflection rate is fallen within a range shown by the following equation: thm (k)$\leqq$nm reflection rate on the test papers on the measuring test device<thm (k + 1)

in which th shows threshold value for the ranking determination, which is predetermined on each of the items to be measured and on each of the ranking, and k shows ranking, for instance, from 1 to 10, and n and m have the same meaning as above.

In the present invention, the correction can be conducted by using only one of the standard value on the calibrate board and the standard value memorized in the memory for the standard value, though accuracy may be somewhat reduced. In this case, the same manner as above can be conducted, except for conducting no correction on the colored standard sheets.

Namely, correction coefficient is obtained, so that the standard values of the white standard sheets are coincided with the measurement values the white standard sheets on the measuring test device, and the slant of the straight line is obtained from the correction coefficients on the both white standard sheets on the measuring test device, and the correction coefficients on each test papers are obtained from this straight line, and each of the correction coefficients is multiplied by the measured values on each of the test papers.

In the above example, measurement is conducted by positioning the reading parts for the test device neat the upper part of the test device, because urine test device is used as the test device. In case of measuring with the use of the test device for measuring components in blood (for instance dry chemistry film, etc.) wherein a blood sample such as blood, plasma and serum is dropped to cause a reaction and measurement of the result is conducted from the opposite side to the dropping surface, however, the desired measurement can be conducted by positioning the reading part for the test device near the bottom surface (reverse) of the test device, as shown in Figure 18. In case of using plasma, serum, etc., measurement from the upper position as in urine test device can be conducted even when a test device for measuring components in blood is used.

According to the present invention, the measuring parts can freely be transported, and only positioning the measuring parts opposite to the test device for the measurement is enough to attain the object, and thus measurement can be conducted without no delay in any places. This is excellent function which has never been attained in conventional test and analysis instruments.

In the analyzer of the present invention, the measuring parts and the control parts may be constructed separately or integratedly, and when those are constructed separately and connected by a lead wire, for instance, with each other, the control parts can be transported with holding in a pocket, and the measurement can be conducted only by positioning the measuring parts opposite to the test device. This is very convenient feature of the present invention.

Advantageous Effect of the Invention

As explained above, according to the test device of the present invention, measurements of many items can be conducted satisfactorily even with a sample containing a small amount of a sample such as an urine, blood, a culture solution of microorganism etc, which has never been possible in known methods, and further the whole items can be measured with one shot dropping of the sample and one cycle of irradiation and measurement of light, and therefore there can be realized such great advantages that the measurements can be conducted in extraordinary high speed which has never been attained in known methods, the size of the analyzer can be made into compact and the test devices can be supplied at remarkably low price as compared with this kinds of conventional test devices.

Additionally in the analyzer using the test devices according to the present invention, it is not necessary, upon distribution and reactions, to transport a distributor or a test device and thus measurement in short time can be conducted and the analyzer itself can be made into compact at low cost.

Also, according to the method for preparation of the present invention, the test devices of the present invention can easily be prepared and manufactured at low cost.

Further, according to the analyzer of present invention, by constructing in such a way that the measuring parts can be transported manually and measurement can be conducted only by positioning the measuring parts opposite to the test device, which has never been known in this kind of conventional analyzer, examination at bed side can easily be conducted, which has been difficult in conventional analyzers, and further regional examination, etc. can easily and accurately be conducted by visual way.

**Claims**

1. A test device for a multi-items test of a sample, characterized in that micro test papers for the multi-items test are held on the bottoms of concave portions in the number required for the multi-items test for one test per the sample, the concave portions being set forth separately with one another by divider walls in such a way that all the test papers for all items for one test are wet by one shot dropping of the sample, and the depth of the concave portions being more than a thickness of the micro test papers.

2. A test device according to Claim 1, wherein said concave portions are provided within the range capable of measuring all test papers without transportation of moving a detecting part or the test device.

3. A test device according to Claim 1, wherein the number of said concave portions is 4 or more, and a diameter of the micro test paper or a distance between the two opposite sides of the micro test paper is 0.5 to 3.0 mm.

4. A test device according to Claim 3, wherein said concave portions are provided within 4 $cm^2$ of an area formed by the utmost external line of the concave portions.

5. A test device according to Claim 1, wherein a recessed part on the upper surface of the micro test papers is constituted to be able to receive one test sample quantity.

6. A test device according to Claim 1, wherein the micro test papers are adhered to the concave portions.

7. A test device according to Claim 1, wherein the sample is an urine or a culture solution of a microorganism and a space is formed between the concave portions and the micro test papers.

**8.** A test device according to Claim 1, wherein said sample is one originating from a living body except an urine, and the concave portions are provided within the range in which all the test papers for all items are wetted by one dropping of the sample from a single distributor.

**9.** A test device according to Claim 8, wherein said sample is one originating from a living body except an urine, and said divider wall and said micro test paper are placed in close contact with each other so that the sample is not permeated from side of the micro test paper.

**10.** A test device according to any of Claim 9, wherein there is equipped with means for impregnating easily the sample dropped on said micro test paper.

**11.** A test device according to Claim 10, wherein surfaces of said micro test papers are subjected to hydrophilic treatment, thereby the sample on the surfaces can more easily be impregnated.

**12.** A test device according to Claim 10, wherein a thin sheet contacting with the reverse surface of said micro test paper is constructed as porous or mesh so as to deflate air through pores to outside atmosphere upon dropping of a sample, so that the sample can more easily be impregnated.

**13.** A test device according to Claim 1, wherein said concave portions for one test per the sample and in addition a concave portion for dropping of the sample are set forth and the both concave portions are connected through.

**14.** A test device according to Claim 13, wherein said concave portion for dropping of the sample is set forth on the central part and said concave portions for one test per the sample are set forth around the concave portion for dropping of the sample.

**15.** A test device according to Claim 13, wherein the base of said concave portion for dropping of the sample on the central part is set forth at higher position than the base of said concave portions for one test per the sample and ditches connecting each the concave portion for dropping the sample and those for one test per the sample to one another are formed in downwardly slant.

**16.** A test device according to Claim 13, wherein a ditch or a hole is set forth around said concave portions for one test per the sample so that an excess amount of the sample overflown from said concave portions is contained in said ditch or hole.

**17.** A method for preparation a test device for a multi-items test of a sample in which micro test papers for measuring items are held within concave portions for the number of one test per the sample for measuring a multi-items provided through a divider wall, said concave portions for one test being provided within the range in which all the test papers for all items are wetted by dropping of one test sample, and the depth of said concave portions are greater than the thickness of said test paper, said method comprising the steps of:

(1) a process of adhering a second sheet material formed with a number of through-holes to a first sheet material, and
(2) a process of adhering a micro test paper on said hole or a part at which said hole is positioned before or after said second sheet material is adhered,
said hole having the depth greater than the thickness of said test paper.

**18.** A method for preparation a test device for a multi-items test of a sample in which micro test papers for measuring items are held within concave portions for the number of one test per the sample for measuring a multi-items provided through a divider wall, said concave portions for one test being provided within the range in which all the test papers for all items are wetted by dropping of one test sample, and the depth of said concave portions are greater than the thickness of said test paper, said method comprising the steps of:

(1) a process of adhering a second sheet material formed with a number of through-holes to a first sheet material,
(2) a process of positioning a micro test paper to said hole or a part at which said hole is positioned before or after said second sheet material is adhered, and

(3) a process of adhering a third sheet material formed with a number of through-holes to the upper part of said second sheet material in such a way that the holes of the third sheet material are lied upon those in the second sheet material.

**19.** A method according to Claim 17, wherein said first sheet material is a tape whose both sides are sticky, and which comprises a process of adhering said second sheet material formed with a number of through-holes to one surface of said tape and adhering a thin sheet to another surface of said tape to form a chip type, a stick type or a slide type test device.

**20.** A method according to Claim 17, wherein the sample is one originating from a living body expect an urine, and which comprises (1) a process of adhering a tape whose both sides are sticky to both surfaces of a second sheet material made of a transparent material, forming a number of through-holes for positioning micro test papers and adhering a first sheet material formed with a number of through-holes for deflating air to one surface of the second sheet material, (2) a process of positioning said micro test papers to said holes or a part at which said holes of the second sheet material is positioned, and (3) a process of adhering a third sheet material formed with a number of through-holes to the other surface of said third sheet material in such a way that the holes of the third sheet material are lied upon those of the second sheet material.

**21.** A method according to Claim 17, wherein said micro test papers are die-cut by many columns having blades on their edges, the die-cut micro test papers are aspirated and held in the columns kept under reduced pressure, said columns are positioned oppositely to said holes or said part at which said holes are positioned of said second sheet material, and the micro test papers are placed in said holes or said part or adhered to said second sheet material of said holes or said parts by increasing the inner pressure of said columns to normal or elevated one.

**22.** A method according to Claim 17, wherein said micro test papers are die-cut by many columns having blades on their edges, test papers obtained by die-cut are pierced by tools having one or plural needles to hold the papers in the needles, said tools are positioned opposite to said holes or said parts at which said holes are positioned of said second sheet material and said test papers are placed in said holes or said parts or adhered to said second sheet material of said holes or said parts by taking out of the test papers from the needles.

**23.** A method according to Claim 17, wherein the above mentioned second sheet material is made of materials treated with a hydrophilic substance or materials whose surface is subjected to hydrophilic treatment.

**24.** An analyzer for detecting components of a sample in a measuring test device which is composed of measuring parts equipped with a detecting part for measuring reflection lights and control parts set forth integrated in or separately from the measuring parts, which is characterized in that there are set forth parts for reading the measuring test device in said measuring parts opposite to the measuring test device and parts for arithmetic in said control parts, which correct, as compared with a standard, a difference in a measuring value caused by a fluctuation of a distance between the measuring test device and the detecting part in each measurement, and said measuring parts are constructed as manually transportable.

**25.** An analyzer according to Claim 24, wherein said measuring test device is an urine test device or a microorganism test device.

**26.** An analyzer according to Claim 24, wherein said measuring test device is a test device for measuring components in a sample originating from a living body except an urine.

**27.** An analyzer according to Claim 24, wherein said measuring parts and said control parts are set forth separately and the both parts are connected by a lead wire or a radio transmission means with each other.

**28.** An analyzer according to Claim 24, wherein said measuring test device is composed of test papers for the items to be tested and white standard parts which take a role as the first standards for correcting the measured values on said test sheets of papers.

**29.** An analyzer according to Claim 28, wherein a calibrate board composed of colored standard sheets having the same color as developed by said test papers and white standard parts, which act as the second standards, are equipped in a position of predetermined distant from the detecting part, or reflection light signal from said colored standard sheets and the white standard parts in the calibrate board is memorized in a memory for a standard value equipped in said control parts and thus memorized standard value is used as the third standard, and the measured values on said test papers are corrected in such a way that the reflection light signals on the white standard parts in said third or second standard become the same with the reflection light signals on white standard parts in said first standard.

30. An analyzer according to Claim 29, wherein said white standard parts are white standard sheets, and said measuring test device is composed in such a way that said test papers are put hold between said both white standard sheets, and said calibrate board is composed in such a way that said colored standard sheets are put hold between said both white standard sheets, and fellow white standard sheets and said test sheets of papers and said colored standard sheets in said calibrate board and said measuring test device are, respectively, positioned oppositely with each other at an interval.

31. An analyzer according to Claim 30, wherein said third standards are divided by each of said second standards to give the first correction coefficient, the second correction coefficients on each of the colored standard sheets are calculated from the slants of straight lines formed by connecting the first correction coefficients of each of the white standard sheets, said second correction coefficients are multiplied by the measured values of each of the colored standard sheets, the standard values of each of the colored standard sheets are divided by thus obtained multiplied values to give the third correction coefficients and said third correction coefficients are multiplied by the measured values of each of the papers to correct the optical systems to the standard values, so that the reflection light signals of said second standards, the white standard sheets, are coincided with said third standards.

32. An analyzer according to Claim 31, wherein the measured values on the reflection lights of each of the both white standard sheets in said measuring test devices are multiplied by said first correction coefficients, the standard values of the white standard sheets in the third standards are divided by thus obtained multiplied values to give the forth correction coefficients, the fifth correction coefficients of each of the colored standard sheets are calculated from the slants of the straight lines formed by connecting each of the forth correction coefficients, and the fifth correction coefficients are multiplied by the measured values of each of the test papers to correct the distance deviations of the optical systems.

33. An analyzer according to Claim 32, wherein the measured values on the both white standard sheets in said third standards are measured and memorized under such conditions as attaining coincidence.

34. An analyzer according to Claim 32, wherein the white standard parts in said measuring test device are made of such a material as repellent almost completely an urine, a culture solution of a microorganism or a blood sample, so that the reflection lights before and after immersing the parts in the urine, the culture solution of the microorganism or the blood sample are not deviated from each other.

35. An analyzer according to Claim 32, wherein said measuring parts are composed of, as the measuring optical systems, lens systems, detection parts and light source parts.

36. An analyzer according to Claim 35, wherein said detection parts are sensors containing photo-receptor elements.

37. An analyzer according to Claim 36, wherein the sensors containing photo-receptor elements are photo-cells, image sensors or CCD (charge-coupled instruments) sensors.

38. An analyzer according to Claim 24, wherein the both white standard sheets in said measuring test devices are constructed in such a way that measurable sounds or indications are developed when certain dose of lights which can be used as data are obtained and can be recognized as definite modes.

F I G. 1

FIG. 2

FIG. 3

FIG. 4

FIG. 5

FIG. 6

FIG. 7

FIG. 8

5'

12'

B — — B

17

FIG. 9

14    17    12'    5'

1

F I G. 1 0

F I G. 1 1

FIG. 12

FIG. 13

FIG. 14

FIG. 15

FIG. 16

〈READING PARTS〉 40 36 〈BODY PARTS〉

24 43

42

| No.100 | 10S | | |
|--------|-----|-----|-----|
| URO | - | GLU | - |
| BLD | +2 | PRO | - |
| BIL | - | pH | 6.0 |
| KET | +1 | SG | 15 |
| ASC | - | NIT | +3 |

41

39

# F I G . 1 7

$\pm$ — n=1

n=2

n=9

$\mp$ — n=10

# F I G . 1 8

21  24

22

26

29

22

30

27

22

28    28

21

24

22

23

22

25''

FIG. A

4'

FIG. B

4'

FIG. C

4'

FIG. D

4'

F I G. E

F I G. F

F I G. G

FIG. H

FIG. I

FIG. J